# EUROPEAN PATENT APPLICATION

(11) **EP 4 502 604 A1**
(43) Date of publication of application: **05.02.2025**
(21) Application number: 23773943.8
(22) Date of filing: 23.03.2023
(51) Int. Cl.: G01N 33/558

(54) **TEST REAGENT PLATE AND METHOD**

(30) Priority: 24.03.2022 CN 202210301315
(71) Applicant: Leadway (HK) Limited, Sheung Wan, Hong Kong (CN)
(72) Inventor: SHANG, Tao, Hangzhou, Zhejiang 310030 (CN); FEI, Fengqin, Hangzhou, Zhejiang 310030 (CN); KONG, Jianxi, Hangzhou, Zhejiang 310030 (CN); ZHENG, Zhenzhen, Hangzhou, Zhejiang 310030 (CN); TONG, Fangli, Hangzhou, Zhejiang 310030 (CN); DU, Yujiao, Hangzhou, Zhejiang 310030 (CN); WU, Silu, Hangzhou, Zhejiang 310030 (CN); CUI, Yaru, Hangzhou, Zhejiang 310030 (CN)
(74) Representative: Gulde & Partner
(86) International application number: PCT/CN2023/083237
(87) International publication number: WO 2023/179696

(57) **Abstract**

The present invention provides a rapid detection reagent plate for biological samples and its preparation method, a method for promoting the release of marker on a lateral flow test strip. The test reagent device (100) includes an upper cover (101), a lower plate (102), and a test strip (1) assembled between the upper cover (101) and the lower plate (102). The test strip (1) includes a sample pad (2), a label binding pad (3), a test pad (4) and an absorbent pad (5) superposed on each other in sequence, the sample pad (2), the label binding pad (3), the test pad (4) and the absorbent pad (5) are all adhered to a bottom card (6), and the label binding pad (3) is treated in advance with a binding pad treatment solution which includes a saccharide, a polymer and a surfactant. By means of pre-treatment with the label binding pad treatment solution, release of the marker on the test strip can be effectively promoted, and the background of the test strip after test is completed is clean, such that an operator can read a test result on the test pad easily.

## Description

### Field of the Invention

The present invention belongs to the technical field of biological testing, in particular to a test strip, a test reagent device and a manufacturing method thereof in the field of rapid immunoassay.

### Background of the Invention

A lateral flow test strip is a common POCT product at present, and it uses the principle of immunochromatography to achieve transfer of a sample on a test strip and obtain a test result. The lateral flow test strip generally includes a bottom card, and a sample pad, a label binding pad (which may also be referred to as a label pad, and generally uses glass fiber as a carrier), a test pad (which generally uses an NC membrane as a carrier) and an absorbent pad (which generally uses an absorbent material such as filter paper) are adhered to the bottom card in a manner of being superposed on each other in sequence from upstream to downstream. The label pad includes labels that can be bound to an analyte, for example, latex labeled with antigens or antibodies, colloidal gold with antigens or antibodies, fluorescent microspheres with antigens or antibodies, etc. The test pad is generally provided with a test line and a control line. According to different reaction principles, as the sample flows on the test strip, the label will be captured and gathered or not captured on the test line. The presence/absence or concentration of the analyte is determined according to the signal of the label, such as a color signal or a fluorescence signal. The control line can be used to determine whether the test strip is valid or to position an instrument when the test result is being read, etc.

Taking the antigen testing of SARS-CoV-2 as an example, the test strip adopts the lateral flow technology. The test strip includes a sample pad, a label binding pad, a test pad and an absorbent pad, which are adhered to a plastic bottom card in a manner of being superposed on each other in sequence. The label binding pad includes an anti-SARS-CoV-2 antibody-latex label, the test line (T line) of the test pad is coated with an anti-SARS-CoV-2 antibody, and the control line (C line) of the test pad is coated with goat-anti-mouse IgG The prepared test strip can also be assembled in the test reagent device for use. Such antigen test strip for SARS-CoV-2 can be used for rapid screening and reassigning management of people, in order to shorten a transmission chain. It has the advantages of being fast, simple and practicable, and can be used as a supplementary means for testing of novel coronavirus pneumonia.

When a sample is tested, a treated sample solution is added to the sample pad of the test strip, the sample on the test pad successively flows past the label binding pad and the test pad, and the excess sample solution is absorbed by the absorbent pad at last. If the sample solution passes by the label binding pad and the latex labels on the label binding pad are released slowly, the following situation often occurs: the latex labels are separated from the sample solution, the aqueous sample solution is subjected to chromatography first through an NC membrane to reach the absorbent pad, and the latex labels are subjected to slow chromatography. This case can cause label complexes (e.g., latex labels) to fail to complete immunochromatography assay (ICA) and reach the absorbent pad within the specified time. These slowly released latex labels have a large quantity of residues on the NC membrane, causing the background color on the NC membrane to darken, which affects the color development of the C and T lines, thus affecting the interpretation result of positive or negative of the sample. Therefore, how to quickly and fully release the labels and complete chromatography synchronously with the sample solution is an urgent problem in the field of rapid immunoassay, especially in the field of rapid immunoassay using the lateral flow technology at present.

### Summary of the Invention

In order to solve the above problems in the prior art, the present invention provides a test strip. The test strip comprises a sample pad, a label binding pad, a test pad, an absorbent pad and a bottom card playing a supporting role superposed on each other in sequence, the sample pad, the label binding pad, the test pad and the absorbent pad are adhered to the bottom card, and the label binding pad contains a binding pad treatment solution which comprises a saccharide, a polymer and a surfactant.

Further, the concentration of the saccharide is 5% to 30% (m/V). Still further, the concentration of the saccharide is 5% to 15% (m/V). Preferably, the saccharide is selected from sucrose or fructose.

Further, the concentration of the polymer is 0.1% to 20% (m/V). Still further, the concentration of the polymer is 0.1% to 10% (m/V). Preferably, the polymer is selected from polyvinylpyrrolidone (PVP) or polyvinyl alcohol.

Further, the concentration of the surfactant is 0.1% to 15% (m/V). Still further, the concentration of the surfactant is 0.1% to 10% (m/V). Preferably, the surfactant is selected from Tween-20 or Silwet L7600.

Further, the binding pad treatment solution further comprises a buffer reagent. The buffer reagent is selected from a phosphate buffer saline.

The present invention also provides a test reagent device, comprising an upper cover, a lower plate, and a test strip assembled between the upper cover and the lower plate, the test strip comprises a sample pad, a label binding pad, a test pad, an absorbent pad and a bottom card superposed on each other in sequence, the sample pad, the label binding pad, the test pad and the absorbent pad are adhered to the bottom card, and the label binding pad contains a binding pad treatment solution which comprises a saccharide, a polymer and a surfactant.

The present invention also provides a preparation method of a test strip, comprising the following steps:
step 1: sticking a test pad to a bottom card, and coating a T-line material and a C-line material to positions of the T line and C line of the test pad;
step 2: preparing a binding pad treatment solution and treating a binding pad with the binding pad treatment solution, and drying;
step 3: treating an immune label on the label binding pad treated in step 2, and drying;
step 4: sticking the label binding pad prepared in step 3 and an absorbent pad to two ends of the test pad, respectively;
step 5: sticking the sample pad to the other end of the label binding pad to prepare a test strip;the binding pad treatment solution comprising a saccharide, a polymer and a surfactant.

The present invention also provides a preparation method of a test reagent device, and on the basis of preparation of the test strip described in the present invention, step 6 is added to install the test strip prepared in step 5 between an upper cover and a lower plate to obtain a test reagent device.

The present invention also provides a method for promoting the release of labels on a lateral flow test strip, the label binding pad of the lateral flow test strip contains a binding pad treatment solution, and the binding pad treatment liquid comprises a saccharide, a polymer, and a surfactant.

In a further preparation method, the concentration of the saccharide is 5% to 30% (m/V). Still further, the concentration of the saccharide is 5% to 15% (m/V). Preferably, the saccharide is selected from sucrose or fructose.

In a further preparation method, the concentration of the polymer is 0.1% to 20% (m/V). Still further, the concentration of the polymer is 0.1% to 10% (m/V). Preferably, the polymer is selected from polyvinylpyrrolidone (PVP) or polyvinyl alcohol.

In a further preparation method, the concentration of the surfactant is 0.1% to 15% (m/V). Still further, the concentration of the surfactant is 0.1% to 10% (m/V). Preferably, the surfactant is selected from Tween-20 or Silwet L7600.

In a further preparation method, the binding pad treatment solution further comprises a buffer reagent. The buffer reagent is selected from a phosphate buffer saline.

The label binding pad of the test strip described in the present invention is treated in advance with the binding pad treatment solution, which can effectively promote the release of labels on the test strip, and the test strip after completion of the testing has a clean background, not affecting the color development of the result on the test pad and not affecting the interpretation of the test result, so that it is easy for an operator to interpret the test result on the test pad. The test strip and the test reagent device described in the present invention are simple to operate and give results rapidly, so that non-professionals also can operate the rapid immunoassay test strip and test reagent device in the present invention to rapidly screen diseases of people, especially screen infectious diseases, for example, to test SARS-CoV-2, etc.

### Brief Description of the Drawings

Fig. 1 is a structure diagram of a test strip.
Fig. 2 is a structure diagram of putting the test strip into a test reagent device.
Fig. 3 is an exploded view of the test reagent device.

### Detailed Description of the Embodiments

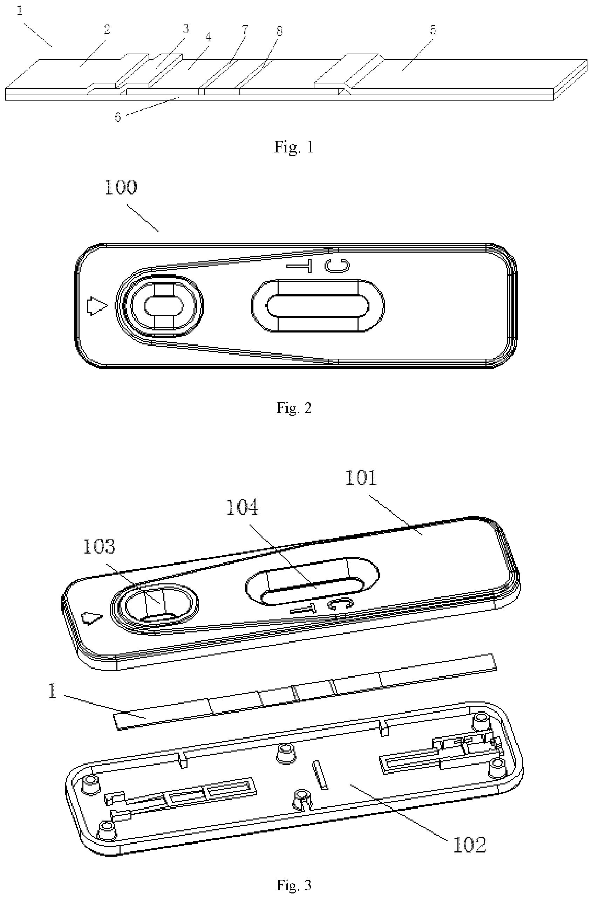

As shown in Fig. 1, the present invention provides a rapid immunoassay test strip 1. The test strip 1 includes a sample pad 2, a label binding pad 3, a test pad 4, an absorbent pad 5, and a bottom card 6 playing a supporting role, which are superposed on each other in sequence. The sample pad, the label binding pad, the test pad, and the absorbent pad are all adhered to the bottom card. The test pad 4 is provided with a test line 7 (T line) and a control line 8 (C line). According to different test items, the label binding pad, the test line and the control line include test reagents adaptive to the test items.

As shown in Figs. 2 and 3, the test strip described in Fig. 1 is installed in a test reagent device 100. The test reagent device includes an upper cover 101 and a lower plate 102 which can be interlocked and fix the test strip 1 in the test reagent device. The upper cover of the test reagent device also includes a sample addition hole 103 and an observation window 104. The sample pad 2 of the test strip is located under the sample addition hole 103. The test pad 4 of the test strip is located under the observation window 104 to facilitate observing the test result.

In addition to containing an immune label, the label binding pad of the test strip described in the present invention also includes a binding pad treatment solution that promotes the release of the immune label from the label binding pad. The binding pad treatment solution includes a saccharide, a polymer and a surfactant.

The saccharide in the binding pad treatment solution is selected from but not limited to sucrose and fructose, the polymer is selected from but not limited to polyvinylpyrrolidone (PVP) and polyvinyl alcohol (PVA), and the surfactant is selected from but not limited to Tween-20 (Tween-20) and Silwet L7600 (S16).

The buffer solution in the binding pad treatment solution is selected from but not limited to phosphate buffer saline.

The treatment method of the label binding pad described in the present invention includes the following steps:
Step 1: mixing a saccharide, a polymer, and a surfactant proportionally in a buffer solution to prepare a binding pad treatment solution.
Step 2: immersing the blank untreated binding pad into the prepared binding pad treatment solution, removing and drying to make the treated binding pad, the material of the binding pad being selected from but not limited to polyester film or glass fiber.
Step 3: treating the immune label on the binding pad prepared in step 2, and drying for later use.

A method for preparing the rapid immunoassay test strip described in the present invention includes the following steps:
Step 1: sticking a test pad 4 to a bottom card 6, and coating a T-line material and a C-line material to positions of the T line and C line of the test pad.
Step 2: sticking the label binding pad prepared by the treatment method of the label binding pad described in the present invention and an absorbent pad to two ends of the test pad, respectively.
Step 3: sticking the sample pad to the other end of the label binding pad to prepare the test strip.

The test strip prepared in step 3 may be installed in the test reagent device 100, or may be directly used for testing, or may also be used after an adhesive is sticked to the sample pad and the absorbent pad, respectively.

### Example 1 Antigen test strip for SARS-CoV-2

Experimental materials:
1. Formulation of the binding pad treatment solution:
   The formulation of the binding pad treatment solution includes a saccharide, a polymer and a surfactant.

The saccharide is selected from sucrose or fructose

The polymer is selected from polyvinylpyrrolidone (PVP) or polyvinyl alcohol (PVA). The surfactant is selected from Tween-20 (Tween-20) or Silwet L7600 (S16).

The formulation also includes a buffer solution, and the buffer solution in this example is selected from phosphate buffer saline (PBS, 0.01mol·L⁻¹, pH 7.4). Material of the binding pad: glass fiber.
2. Immune label: anti-SARS-CoV-2 antibody-latex label.
3. Material of the bottom card: plastic, for example, selected from but not limited to PVC material.
4. Material of the test pad: NC membrane.
5. C-line solution: goat-anti-mouse IgG
6. T-line solution: anti-SARS-CoV-2 antibody.
7. Material of the absorbent pad: filter paper.
8. Formulation of the reagent on the sample pad: phosphate buffer saline, Tween-20, and casein. Material of the sample pad: glass fiber.
9. Sample extracting solution: PBS, 0.01mol·L⁻¹, pH 7.4. A sample collected with a nasal swab is placed in the sample extracting solution to obtain the test sample.

### Experimental method:

1. Sucrose, fructose, PVP, PVA, Tween-20 and Silwet L7600 (S16) are prepared in different proportions in PBS to form the corresponding binding pad treatment solutions. The blank binding pad is immersed in the solution for full soaking, and then is removed and dried in an oven at 55°C to make the treated binding pad. The formulation of the binding pad treatment solution is as shown in Table 1:

**Table 1 Formulation of the binding pad treatment solution**

| **Number of Formulation** | Working concentration of chemical (m/V) | | | | | |
|---|---|---|---|---|---|---|
| | **Sucrose** | **Fructose** | **PVP** | **PVA** | **Tween-20** | **S16** |
| **1** | / | / | / | / | / | / |
| **2** | / | 20% | 15% | / | 10% | / |
| **3** | | 20% | / | 15% | 10% | / |
| **4** | 20% | / | 15% | / | / | 10% |
| **5** | 20% | / | / | 15% | / | 10% |
| **6** | 2% | / | 0.05% | / | 0.05% | / |
| **7** | | | | | 0.1% | |
| **8** | | | 0.1% | | 0.05% | |
| **9** | | | | | 0.1% | |
| **10** | 5% | / | 0.1% | | 0.1% | / |
| **11** | | | | | 10% | |
| **12** | | | 10% | | 0.1% | |
| **13** | | | | | 10% | |
| **14** | 15% | / | 10% | | 10% | / |
| **15** | | | | | 15% | |
| **16** | | | 20% | | 10% | |
| **17** | | | | | 15% | |
| **18** | 30% | / | 20% | | 15% | / |
| **19** | | | | | 20% | |
| **20** | | | 25% | | 15% | |
| **21** | | | | | 20% | |
| **22** | 35% | / | 25% | | 20% | / |
| **23** | 2% | / | / | 0.05% | 0.05% | / |
| **24** | | | | | 0.1% | |
| **25** | | | | 0.1% | 0.05% | |
| **26** | | | | | 0.1% | |
| **27** | 5% | / | | 0.1% | 0.1% | / |
| **28** | | | | | 10% | |
| **29** | | | | 10% | 0.1% | |
| **30** | | | | | 10% | |
| **31** | 15% | / | | 10% | 10% | / |
| **32** | | | | | 15% | |
| **33** | | | | 20% | 10% | |
| **34** | | | | | 15% | |
| **35** | 30% | / | | 20% | 15% | / |
| **36** | | | | | 20% | |
| **37** | | | | 25% | 15% | |
| **38** | | | | | 20% | |
| **39** | 35% | / | | 25% | 20% | / |
| **40** | 5% | / | / | / | 0.1% | / |
| **41** | 30% | / | / | / | 15% | / |
| **42** | / | / | 0.1% | / | 0.1% | / |
| **43** | / | / | 20% | / | 15% | / |
| **44** | / | / | / | 0.1% | 0.1% | / |
| **45** | / | / | / | 20% | 15% | / |
| **46** | 5% | / | 0.1% | / | / | / |
| **47** | 30% | / | 20% | / | / | / |
| **48** | 5% | / | / | 0.1% | / | / |
| **49** | 30% | / | / | 20% | / | / |

| | | | | | | |
|---|---|---|---|---|---|---|
| * (m/V) represents grams of a solute dissolved in per 100ml of a solvent | | | | | | |

2. The anti-SARS-CoV-2 antibody-latex label was uniformly dispensed onto the treated binding pad using a gold sprayer, and the binding pad was dried in an oven at 37°C.
3. The dried binding pad bound with the anti-SARS-CoV-2 antibody-latex label, the NC membrane coated with goat-anti-mouse IgG and the anti-SARS-CoV-2 antibody (adhered to the bottom card), the sample pad, and the absorbent pad were assembled in the same manner as the conventional test strip, i.e., the sample pad, the label binding pad, the test pad, and the absorbent pad were superposed on each other in sequence and sticked onto the bottom card to make the test strip, and the test strip was installed in the test reagent device to prepare the test reagent device.
4. The sample was treated with an extracting solution, and sample addition test was performed to observe the ICA situation of the latex on the test strip. It was regarded as normal ICA in the case that the anti-SARS-CoV-2 antibody-latex label was fully released, the background of ICA was clean (i.e., the background of the NC membrane was clean), and the latex label was not separated from the aqueous solution. It was regarded as abnormal ICA in the case that the latex label was slowly released and separated from the aqueous solution, the aqueous solution was chromatography to reach the absorbent pad at first, while the latex label was subjected to slow chromatography and failed to complete ICA within the specified time, residual latex label was left on the NC membrane, and the background of ICA was dark. 100 tests were repeated for each formulation, and experimental data were collected. Experimental results are shown in Table 2.

**Table 2 Experimental results:**

| **Formulation Number** | **Experimental result** | | **Formulation Number** | **Experimental result** | |
|---|---|---|---|---|---|
| | Normal ICA (%) | Abnormal ICA (%) | | Normal ICA (%) | Abnormal ICA (%) |
| **1** | 79 | 21 | **26** | 80 | 20 |
| **2** | 97 | 3 | **27** | 100 | 0 |
| **3** | 98 | 2 | **28** | 100 | 0 |
| **4** | 96 | 4 | **29** | 100 | 0 |
| **5** | 99 | 1 | **30** | 100 | 0 |
| **6** | 89 | 11 | **31** | 100 | 0 |
| **7** | 80 | 20 | **32** | 97 | 3 |
| **8** | 81 | 19 | **33** | 98 | 2 |
| **9** | 89 | 11 | **34** | 97 | 3 |
| **10** | 100 | 0 | **35** | 96 | 4 |
| **11** | 100 | 0 | **36** | 77 | 23 |
| **12** | 100 | 0 | **37** | 75 | 25 |
| **13** | 100 | 0 | **38** | 82 | 18 |
| **14** | 100 | 0 | **39** | 76 | 24 |
| **15** | 97 | 3 | **40** | 84 | 16 |
| **16** | 98 | 2 | **41** | 81 | 19 |
| **17** | 97 | 3 | **42** | 79 | 21 |
| **18** | 98 | 2 | **43** | 84 | 16 |
| **19** | 75 | 25 | **44** | 75 | 25 |
| **20** | 84 | 16 | **45** | 80 | 20 |
| **21** | 76 | 24 | **46** | 75 | 25 |
| **22** | 84 | 16 | **47** | 84 | 16 |
| **23** | 81 | 19 | **48** | 74 | 26 |
| **24** | 75 | 25 | **49** | 75 | 25 |
| **25** | 84 | 16 | / | / | / |

### Analysis:

Upon analysis, in the formulation of the pretreatment reagent of the label binding pad on the antigen test strip for SARS-CoV-2 in this example, the label binding pad was treated with sucrose, fructose, PVP, PVA, Tween-20, and Silwet L7600 (S16) according to the ranges of working concentrations in Table 3, and the test strip was prepared. The test strip showed normal ICA (the rate of normal ICA ≥95%) during the test. Normal ICA means that the latex label is fully released, the background of the test pad is clean, and the latex label is not separated from the aqueous solution.

**Table 3**

| Chemicals | Working concentration |
|---|---|
| Saccharides: sucrose or fructose | 5%-30% (m/V) |
| Polymer: polyvinylpyrrolidone (PVP) or polyvinyl alcohol (PVA) | 0.1%-20% (m/V) |
| Surfactant: Tween-20 (Tween-20) or Silwet L7600 (S16) | 0.1%-15% (m/V) |

Upon analysis, in the formulation of the pretreatment reagent of the label binding pad on the antigen test strip for SARS-CoV-2 in this example, the label binding pad was treated with sucrose, fructose, PVP, PVA, Tween-20, and Silwet L7600 (S16) according to the ranges of working concentrations in Table 4, and the test strip was prepared. The test strip showed normal ICA (the rate of normal ICA could reach 100%) during the test.

**Table 4**

| Chemicals | Working concentration |
|---|---|
| Saccharides: sucrose or fructose | 5%-15% (m/V) |
| Polymer: polyvinylpyrrolidone (PVP) or polyvinyl alcohol (PVA) | 0.1%-10% (m/V) |
| Surfactant: Tween-20 (Tween-20) or Silwet L7600(S16) | 0.1%-10% (m/V) |

## Claims

1. A test reagent device, comprising an upper cover, a lower plate, and a test strip assembled between the upper cover and the lower plate, the test strip comprising a sample pad, a label binding pad, a test pad, an absorbent pad and a bottom card superposed on each other in sequence, the sample pad, the label binding pad, the test pad and the absorbent pad being adhered to a bottom card, wherein the label binding pad contains a binding pad treatment solution which comprises a saccharide, a polymer and a surfactant.

2. The test reagent device according to claim 1, wherein the concentration of the saccharide is 5% to 30% (m/V).

3. The test reagent device according to claim 2, wherein the concentration of the saccharide is 5% to 15% (m/V).

4. The test reagent device according to claim 1, wherein the concentration of the polymer is 0.1% to 20% (m/V).

5. The test reagent device according to claim 4, wherein the concentration of the polymer is 0.1% to 10% (m/V).

6. The test reagent device according to claim 1, wherein the concentration of the surfactant is 0.1% to 15% (m/V).

7. The test reagent device according to claim 6, wherein the concentration of the surfactant is 0.1% to 10% (m/V).

8. The test reagent device according to one of claims 1 to 7, wherein the saccharide is selected from sucrose or fructose; and/or the polymer is selected from polyvinylpyrrolidone or polyvinyl alcohol; and/or the surfactant is selected from Tween-20 or Silwet L7600.

9. A preparation method of a test reagent device, comprising the following steps:
step 1: sticking a test pad to a bottom card, and coating a T-line material and a C-line material to positions of the T line and C line of the test pad;
step 2: preparing a binding pad treatment solution and treating a label binding pad with the binding pad treatment solution, and drying;
step 3: treating an immune label on the label binding pad treated in step 2, and drying;
step 4: sticking the label binding pad prepared in step 3 and an absorbent pad to two ends of the test pad, respectively;
step 5: sticking the sample pad to the other end of the label binding pad to prepare a test strip; and
step 6: installing the test strip prepared in step 5 between an upper cover and a lower plate to obtain a test reagent device,
the binding pad treatment solution comprising a saccharide, a polymer and a surfactant.

10. The preparation method according to claim 9, wherein the concentration of the saccharide is 5% to 30% (m/V); the concentration of the polymer is 0.1% to 10% (m/V); and the concentration of the surfactant is 0.1% to 15% (m/V).

11. A method for promoting the release of labels on a lateral flow test strip, wherein the label binding pad of the lateral flow test strip contains a binding pad treatment solution which comprises a saccharide, a polymer, and a surfactant.

12. The method according to claim 11, wherein the concentration of the saccharide is 5% to 30% (m/V).

13. The method according to claim 12, wherein the concentration of the saccharide is 5%-15% (m/V).

14. The method according to claim 11, wherein the concentration of the polymer is 0.1% to 20% (m/V).

15. The method according to claim 14, wherein the concentration of the polymer is 0.1%-10% (m/V).

16. The method according to claim 11, wherein the concentration of the surfactant is 0.1% to 15% (m/V).

17. The method according to claim 16, wherein the concentration of the surfactant is 0.1% to 10% (m/V).

18. The method according to one of claims 11 to 17, wherein the saccharide is selected from sucrose or fructose; and/or the polymer is selected from polyvinylpyrrolidone or polyvinyl alcohol; and/or the surfactant is selected from Tween-20 or Silwet L7600.
